# FASCICULE DE BREVET EUROPEEN

(11) **EP 1 238 056 B1**
(45) Date de publication et mention de la délivrance du brevet: **02.01.2008**
(21) Numéro de dépôt: 00993415.9
(22) Date de dépôt: 12.12.2000
(51) Int. Cl.: C12N 1/20, C12N 5/06, C07K 16/12, G01N 33/53

(54) **BACTERIE RICKETTSIA PULICIS METHODE DE DIAGNOSTIC SEROLOGIQUE**
VERFAHREN ZUR DIAGNOSE VON RICKETTSIA PULICIS
SEROLOGICAL DIAGNOSTIC METHOD WITH i RICKETTSIA PULICIS /i BACTERIUM

(30) Priorité: 14.12.1999 FR 9915777
(43) Date de publication de la demande: 11.09.2002
(73) Titulaire: Université de la Méditerranée, Aix-Marseille II, 13284 Marseille Cedex 07 (FR)
(72) Inventeur: RAOULT, Didier, F-13008 Marseille (FR); LA SCOLA, Bernard, F-13790 Rousset (FR)
(74) Mandataire: Domange, Maxime
(86) Numéro de dépôt international: PCT/FR2000/003475
(87) Numéro de publication internationale: WO 2001/044438

(56) Documents cités:
- WO-A-99/42479
- US-A- 4 447 537
- RADULOVIC ET AL.: "Isolation, cultivation, and partial characterization of the ELB agent associated with cat fleas" INFECTION AND IMMUNITY, vol. 63, no. 12, décembre 1995 (1995-12), pages 4826-4829, XP002145965 cité dans la demande
- HIGGINS ET AL.: "Rickettsia felis: a new species of pathogenic Rickettsia isolated from cat fleas" JOURNAL OF CLINICAL MICROBIOLOGY, vol. 34, no. 3, mars 1996 (1996-03), pages 671-674, XP002145966 cité dans la demande

## Description

La présente d'invention concerne le domaine microbiologique, notamment le domaine du diagnostic microbiologique. Plus précisément l'invention concerne une méthode concernant l'isolement d'une nouvelle bactérie provisoirement nommée comme *«Rickettsia pulicis»* ainsi que l'utilisation de la souche pour la réalisation d'un test sérologique.

L'invention concerne en effet une méthode pour le diagnostic sérologique *in vitro* des infections à *«Rickettsia pulicis»* ainsi qu'un dispositif pour la mise en oeuvre de cette méthode. L'invention concerne également une trousse pour la détection *in vitro* de la bactérie.

Une bactérie avait été détectée mais non isolée dans des puces, aux Etats Unis par des réactions indirectes la coloration par la méthode de Gimenez qui colore les Rickettsies, par des anticorps dirigés contre les Rickettsies et par la mise en évidence de séquences de gènes spécifiques de rickettsie pour cette bactérie. Elle avait été nommée à cette époque agent ELB [Adams JR., Am. J. Trop. Med Hyg.,1990; 43 :400-409]. Des gènes communs à l'ensemble des Rickettsies ont pu être amplifiés chez cette bactérie et séquencés et montrer que cette bactérie était originale. Cette bactérie est considérée comme étant un des éléments expliquant la très grande fréquence de Rickettsioses à puce en Californie [Schriefer ME., J. Med. Entomol., 1994; 31 :681-5]. Une observation [Schriefer ME., J. Clin. Microbiol., 1994; 32 :949-54] a été rapportée d'infection chez l'homme liée à cette bactérie. Cette bactérie a fait l'objet de nombreuses tentatives de culture jusqu'à ce jour sans succès. Les tentatives de culture ont été réalisées sur animaux (souris) puis sur culture cellulaire (cellules VERO et cellule L229) [Higgins JA., J. Clin. Microbiol., 1996; 34 :671-4 ; Radulovic S., Infect. Immun., 1995; 63 :4826-9 ; Radulovic S., Antimicrobial. Agent. Chemother., 1995 ; 39 :2564-6]. Ces trois publications ont rapporté la culture de la bactérie en question qui a été dénommée *Rickettsia felis.* Ces publications n'ont cependant pas été confirmées et aucune souche n'est disponible dans les laboratoires qui ont prétendu avoir isolé cette bactérie. Les envois réalisés par ces laboratoires ont comporté soit des prélèvements dépourvus de toutes rickettsie soit des prélèvements contaminés avec une autre rickettsie, *Rickettsia typhi.* Les analyses par SDS PAGE révèlent que la souche ainsi isolée par ces auteurs qui a été nommée *R. felis* était en fait *R. typhi* et en tout état de cause ne correspondait pas à l'agent ELB. C'est pourquoi la bactérie selon la présente invention qui est en réalité le premier isolat de l'ELB agent a été dénommée provisoirement *«Rickettsia pulicis».*

Il n'existait donc avant la présente invention aucune possibilité de réaliser une réaction sérologique et par ailleurs aucun isolat de cette bactérie n'était disponible dans aucune collection ni aucun laboratoire. En revanche, de nombreuses puces sont infectées naturellement et il existe des collections de puces qui sont infectées à 100% du fait de la transmission systématique de cette bactérie de la mère à sa descendance.

Les inventeurs ont mis au point une méthode spécifique et originale pour la culture de la bactérie *«Rickettsia pulicis».*

Les bactéries du genre *Rickettsia* sont usuellement cultivées à 37°C pour les bactéries du groupe typhus et à 32°C pour les bactéries du groupe boutonneux [Weiss E. Annu. Rev.Microbiol.1982 ; 36 ; 345-70].

Les tentatives de culture sur cellules de mammifères VERO L929, HEL et MRC5 ou d'oiseaux, à 37° et à 32° se sont montrées inefficaces.

Les inventeurs ont découvert que de façon surprenante, la culture des cellules hôtes doit être réalisée à une température inférieure à 30°C, de préférence 25 à 30°C, de préférence encore à 28°C.

Les inventeurs ont réalisé l'inoculation de broyat de puces sur des cellules de *Xenopus laevis* de la lignée XTC-2 cultivées à 28°C. La bactérie est obtenue après 14 à 28 jours de culture; 19/20 des puces qui étaient positives par détection génomique (PCR) ont été cultivées avec succès par cette méthode qui a donc un ratio de succès de 95%. La bactérie de référence isolée (URRWFXCal2) a été établie et actuellement 10 passages ont été obtenus. La bactérie isolée présente les caractéristiques génétiques décrites pour l'agent ELB en ce qui concerne les gènes de la cytrate synthase et de l'ARN 16S (respectivement accès Gen Bank U 33922 et L28944).

La présente invention est donc relative à cette bactérie *Rickettsia pulicis* correspondant à l'agent ELB ainsi isolée qui est établie en culture.

Par "établie en culture", on entend que la bactérie est obtenue de manière reproductible et se multiplie au cours du temps par repiquages successifs sur culture de cellules.

Plus particulièrement la présente invention a pour objet une bactérie *Rickettsia pulicis* isolée et établie en culture sur culture cellulaire à température de 25 à 30°, de préférence 28°C.

Plus particulièrement encore, la bactérie *Rickettsia pulicis* selon l'invention est cultivée sur cellule de *Xenopus laevis,* lignée XTC-2.

Dans un mode de réalisation, la méthode d'isolement selon l'invention comprend les étapes suivantes :
1) on réalise une culture de cellules de préférence de cellules de *Xenopus laevis* lignée XTC-2, à la température de culture de 25 à 30°C de préférence 28°C, et
2) les bactéries sont inoculées par centrifugation du broyat de puces ou tout autre prélèvement d'origine humaine ou animale infecté par lesdites bactéries sur la couche de cellules en culture de l'étape 1, et incubation à 25 à 30°C, de préférence 28°C, jusqu'à détection d'une croissance bactérienne,
3) les bactéries sont établies en culture par repiquages successifs sur des cellules en culture.

Les bactéries peuvent être purifiées par centrifugation du surnageant de culture des cellules de l'étape 3 visée ci-dessus.

La présente invention a également pour objet la détection d'un anticorps spécifique d'une immunoglobuline humaine reconnaissant la dite bactérie, de préférence IgG, IgM ou IgA, et plus particulièrement une immunoglobuline animale, notamment une immunoglobuline anti-humaine de chèvre.

La présente invention a également pour objet une culture cellulaire d'une bactérie selon l'invention et plus particulièrement des cellules de *Xenopus laevis,* de préférence des cellules XTC-2.

Une bactérie selon l'invention a été déposée à la CNCM (Collection Nationale de Culture de Micro-organismes de l'Institut Pasteur-Paris) le 8 décembre 1999 sous le n° d'enregistrement I-2363, identifiée comme *Rickettsia pulicis* (URRWFX cal 2) sous forme de culture cellulaire de cellules XTC-2 infectées. Une culture de cellules saines XTC-2 (non infectées) a été déposée à la CNCM le 8 décembre 1999 sous le n° I-2364.

La présente invention est aussi relative à l'utilisation d'une bactérie, d'une culture ou d'un anticorps spécifique selon l'invention, dans une méthode de diagnostic *in vitro* de maladies liées à des infections par la bactérie *Rickettsia pulicis,* ainsi qu'à une méthode pour le diagnostic sérologique de l'infection à bactérie *Rickettsia pulicis* selon l'invention, qui comprend la mise en contact du sérum ou de tout autre liquide biologique d'un patient avec ladite bactérie et la détection d'une réaction immunologique.

Plus particulièrement, la présente invention a pour objet une méthode de diagnostic sérologique in vitro des infections à *Rickettsia pulicis,* dans laquelle on met en contact la bactérie selon l'invention, ou une culture selon l'invention, avec un échantillon provenant du patient consistant dans un sérum, un fluide biologique, des puces ou un prélèvement humain.

La méthode selon l'invention comprend l'étape consistant essentiellement à détecter une réaction immunologique entre un anticorps spécifique d'une immunoglobuline selon l'invention reconnaissant ladite bactérie et une dite immunoglobuline humaine reconnaissant ladite bactérie.

Dans un mode de réalisation, la méthode de diagnostic selon l'invention comprend :
- le dépôt dans ou sur un support solide d'une solution de bactérie selon l'invention, notamment de 0,5 à 5 µl, de préférence 1 µl de ladite solution contenant ladite bactérie ;
- l'introduction dans ou sur ledit support du sérum ou du fluide biologique à tester dilué;
- l'introduction dans ou sur le support d'une solution d'un anticorps marqué, notamment une immunoglobuline anti-humaine animale spécifique de l'immunoglobuline humaine de type IgG, IgM ou IgA, reconnaissant ladite bactérie ;
- l'observation d'une période d'incubation ;
- le rinçage éventuel du support solide et
- la détection proprement dite de la réaction immunologique entre un anticorps humain reconnaissant ladite bactérie et ladite immunoglobuline anti-humaine.

Comme support solide, on peut utiliser tout dispositif adapté à la manipulation de suspensions cellulaires et bactériennes et notamment des tubes, des lames de verre, des tubes Bijoux ou des plaques rigides de microtitrage en polyéthylène, polystyrène, chlorure de polyvinyle ou nitrocellulose comportant des micropuits, les lames de verre étant préférées.

L'anticorps humain détecté est une immunoglobuline, notamment de type G, M ou A, spécifique de la bactérie selon l'invention.

Avantageusement, la méthode de diagnostic de l'invention met en oeuvre un dosage immunoenzymatique de type ELISA ou un dosage immunofluorescent.

Comme type de marquage de l'immunoglobuline anti-humaine, on utilise donc avantageusement un marquage enzymatique, radioactif ou fluorescent, ce dernier type de marquage étant préféré.

L'expression « marquage fluorescent » signifie que l'anticorps a été rendu fluorescent par couplage ou complexation avec un agent fluorescent approprié tel que l'iso(thio)cyanate de fluorescéine.

L'expression « marquage radioactif » signifie que l'anticorps porte un isotope radioactif permettant de le doser par comptage de la radioactivité qui lui est associée, l'isotope pouvant être porté soit sur un élément de la structure de l'anticorps, par exemple les résidus de tyrosine constitutifs, soit sur un radical approprié qui lui a été fixé.

L'expression « marquage enzymatique » signifie que l'anticorps spécifique est couplé ou complexé à une enzyme qui, associée à l'emploi de réactifs appropriés, permet une mesure quantitative de cet anticorps spécifique.

Le substrat et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant l'échantillon testé et qui fait l'objet, soit de la mesure finale spectrophotométrique ou fluorimétrique, respectivement, soit d'une évaluation à l'oeil ; éventuellement par rapport à une gamme de teintes étalonnées,
- ou bien une substance colorée insoluble qui se dépose sur l'échantillon testé et qui peut faire l'objet, soit d'une mesure photométrique par réflexion, soit d'une évaluation à l'oeil, éventuellement par rapport à une gamme de teintes étalonnées.

Lorsque l'on utilise un anticorps rendu fluorescent, la fluorescence associée à l'échantillon testé est lue directement sur un appareil approprié.

Lorsqu'on utilise une sonde radioactive, comme par exemple l'iode 125, la radioactivité associée à l'échantillon testé est complétée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Lorsque l'on utilise une enzyme sur l'anticorps spécifique, l'apparition d'un produit coloré ou fluorescent est obtenue en ajoutant une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission, ou en réflexion ou fluorimètre respectivement. Alternativement, on peut aussi évaluer à l'oeil la coloration obtenue, en s'aidant éventuellement d'une gamme de solutions colorées étalonnées.

En utilisant comme enzyme la phosphatase alcaline, le couplage de cette enzyme avec l'anticorps spécifique est effectué selon la méthode proposée par Boehringer Mannheim-Biochemica. Les substrats préférentiels de cette enzyme sont le paranitrophénylphosphate pour une lecture fluorométrique ou le bromo-5 chloro-4-umbelliféryl phosphate pour une lecture fluorométrique ou le bromo-5 chloro-4 indolyl-6 phosphate pour obtenir un produit de réaction coloré insoluble. On peut de même utiliser comme enzyme la β-galactosidase dont les substrats préférentiels sont l'orthonitrophényl β-D-galactropyranoside ou le méthyl-4 umbelliféryl β-D-galactopyranoside.

Préférentiellement, on peut coupler les anticorps spécifiques à la péroxydase. Dans ce cas ; le procédé de couplage est dérivé de celui décrit par M.B. Wilson et P.K. Nakane in Immunofluorescence and related staining techniques, W. Knapp, K. Kolubar, G. Wicks ed. Elsevier/North Holland. Amsterdam 1978, p. 215-224.

Les réactifs utilisés pour révéler la péroxydase conjuguée aux anticorps spécifiques contiennent de l'eau oxygénée, sustrat de l'enzyme, et un chromogène approprié par exemple de l'orthophénylénediamine ou l'acide azino-2-2' bis (éthyl-3 thiazoline sulfonique-6) ou ABTS pour obtenir un produit final de réaction coloré et soluble dans le milieu ou bien la diamino-3,3' benzidine ou l'amino-3 éthyl-9 carbazole ou le chloro-4 α-naphtol pour obtenir un produit final de réaction insoluble, ou bien l'acide parahydroxyphényl propionique pour obtenir un produit de réaction fluorescent soluble dans le milieu.

Un autre mode de réalisation de l'invention est l'utilisation d'anticorps spécifiques couplés à l'acétylcholinestérase.

L'acétylcholinestérase est couplée à l'anticorps en utilisant préférentiellement un procédé dérivé de celui décrit dans le brevet français n° 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène reconnu par le conjugué à l'acéthylcholinestérase est réalisée de préfence selon la technique bien connue qui emploie l'acéthylthiocholine comme substrat de l'enzyme et le réactif d'Ellman, ou acide dithio-5,5' nitro-2 benzoïque comme chromogène, selon toute variante adaptée au cas examiné, par exemple celle décrite par Pradelles et al., dans Anal. Chem. 1985, 57 :1170-1173.

Les chromogènes cités sont utilisés tels quels ou sous forme de sels solubles dans l'eau.

La méthode de diagnostic sérologique de l'invention est adaptée à une utilisation dans des laboratoires de biologie et/ou d'anatomopathologie. Pour ce faire, on propose un dispositif pour la mise en oeuvre de cette méthode, qui comprend un support solide sur ou dans lequel on a déposé une solution contenant la bactérie selon l'invention, telle que définie précédemment.

L'invention est aussi relative selon un autre aspect à une trousse pour la détection *in vitro* de *«Rickettsia pulicis».* Cette trousse comprend comme composants :
- une solution contenant « *Rickettsia pulicis »* selon l'invention, isolée et établie comme décrit ci-dessus, à titre de témoin positif;
- une solution contenant un anticorps spécifique reconnaissant la bactérie selon l'invention, et/ou une solution contenant un anticorps spécifique d'une immunoglobuline humaine reconnaissant la bactérie selon l'invention, de préférence marqué ;
- éventuellement, une solution de lavage.

L'anticorps spécifique utilisé dans la trousse de l'invention est avantageusement marqué par une sonde radioactive, une enzyme ou un agent fluorescent.

Lorsque l'anticorps spécifique est marqué par un enzyme, la trousse comprend en outre le substrat de l'enzyme et un ou plusieurs réactifs pour visualiser l'activité de l'enzyme.

Lorsque l'anticorps spécifique est marqué par un agent fluorescent, on utilise de préférence l'iso(thio)cyanate de fluorescéine.

Selon un mode de réalisation préféré de l'invention, on utilise comme anticorps spécifique une immunoglobuline, en particulier une immunoglobuline de souris.

L'invention sera mieux comprise à l'aide de l'exposé ci-après, divisé en sections, qui concernent des expériences effectuées dans le but de réaliser l'invention, et qui sont données à titre purement illustratif.

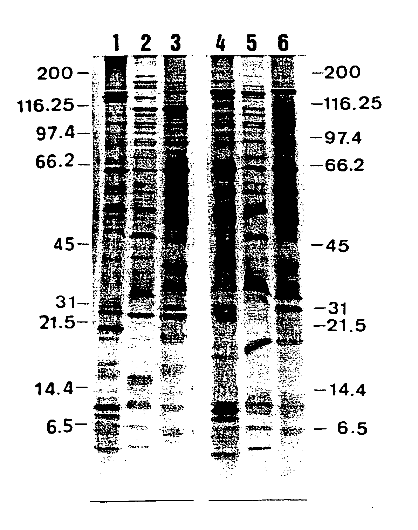

La figure 1 représente le profil protéique des bactéries *R. conorii, R. pulicis* et *R. thyphi* testées à l'exemple 4.

### Exemple 1

### Procédure d'isolement de R. pulicis sur cellules XTC

**1- Primoisolement.** Le primo isolement a été réalisé par la technique de centrifugation sur tubes bijoux inoculés avec la lignée de cellules de *Xenopus laevis,* lignée XTC-2 [Pudney M, Varma MRG, Leake CJ. Establihment of a cell line (XTC-2) from the south african clawed toad Xenopus laevis. Experimentia. 29 :466-467]. Ces cellules sont cultivées sur du milieu Leibowitz-15 avec L-glutamine et L-amino-acides (Gibco, Gaithersburg, MD) additioné de 5% de sérum de veau foetal (Gibco) et de 2% de tryptose phosphate (Gibco). Les tubes bijoux (Sterilin-Felthan-England, 3.7 ml) comportant une lamelle support de 12mm de diamètre sont inoculées avec 1 ml de milieu de culture contenant environ 50 000 cellules sont incubées à 28°C pendant 24 à48 heures de façon à obtenir un tapis de cellules sous-confluentes. L'isolement de la bactérie a été tenté sur 2 lots de puces de chat (*Ctenocephalides felis*)*.* Chaque lot comportait 50 puces. Les puces ont été groupées en 20 groupes de 5 puces (groupes California 1 à 10 et Pete 1 à 10). Dans chaque groupe, les puces ont été décontaminées par une immersion de 5 min. dans de l'alcool méthylique à 70% iodée à 0.2%. Les puces ont ensuite été rincées dans de l'eau distillée, puis congelées dans de l'azote liquide avant d'être broyées. Les puces écrasées ont ensuite été reprise avec 0,8 ml de milieu de culture et le broyat a été utilisée pour inoculer les tubes bijoux (1 par broyat). Ces tubes ont ensuite été centrifugés à 700 X g pendant 1 heure à 22°C. Le surnageant a ensuite été retiré, les tapis ont été lavés deux fois par du tampon PBS stérile, puis incubés à 28°C avec 1 ml de milieu additionné de cotrimoxazole à une concentration finale de 4 µg/ml. Chaque semaine, le surnageant a été remplacé par du milieu de culture frais. Le surnageant retiré a été utilisé pour réaliser des cytocentrifugations pour coloration de Gimenez [Gimenez DF. 1964. Staining rickettsiae in yolk-sac cultures. Stain Technol. 39 :135-140]. Quand la coloration de Gimenez a permis de détecter des bactéries intracellulaires (entre J15 et J30 après l'inoculation), les cellules ont été décollées de la lamelle et inoculées sur un tapis cellulaire sous-confluent dans une boite de culture de 25-cm² avec 5 ml de milieu de culture et incubées à 28°C. Le surnageant a été utilisé pour la détection de *Rickettsia* sp. par amplification et séquençage du gène de la citrate synthase. A l'exception du groupe California 1, tous les groupes étaient positifs.

**2- Propagation de l'isolat.** Le taux d'infection dans les boîtes de cultures cellulaires a ensuite été contrôlé tous les 2 jours par réalisation d'une coloration de Gimenez sur les cellules infectées du surnageant. Lorsque le taux d'infection a été maximal, les cultures cellulaires ont été repiquées sur des boîtes de culture de cellules XTC-2 saines sous-confluentes de 174 cm² avec 30 ml de milieu de culture. Pour la production massive de la souche California 2 (URRWFXCal2) nous avons utilisé une technique de multiplication des cellules infectées par trypsination.Cinq à six jours après infection des cellules XTC-2 dans un boite de culture de 174 cm², lorsque le taux d'infection des cellules était supérieur à 80%, le surnageant de culture a été enlevé, le tapis cellulaire a été lavé par du Rinaldini puis incubé 5 minutes à 37°C avec de la trypsine à 0.05% (Gibco). Les cellules infectées ont été remises en suspension dans 150 ml de milieu de culture. Les cellules infectées ainsi diluées ont été réparties dans 5 boîtes de culture de 174 cm² à raison de 30 ml par boîte. La même procédure a été effectuée pour chaque boîte tous les 5 jours afin de produire l'équivalent de 200 boites de culture de 174 cm² afin d'obtenir du matériel en quantité suffisante pour les études ultérieures. L'absence de contaminants dans les cultures cellulaires a été vérifié tout au long de la production par mise en culture systématique des boîtes de culture récoltées sur gélose au sang et gélose trypticase soja.

**3- Microscopie électronique.** Des cellules infectées, inoculées 8 jours avant par la bactérie, ont été récoltées puis préparées pour l'étude en microscopie électronique. Les cellules ont été fixées dans une solution de glutaraldehyde à 2.5% en tampon PBS 0.15 M (Biomérieux, Marcy l'étoile, France) pendant 1 h à 4°C. Les cellules ont été rincées une nuit dans le même tampon puis fixées 1 h à température ambiante dans du tetroxyde d'osmium en tampon PBS 0.15 M. La déshydratation a été réalisée par rinçages successifs dans des solutions d'acétone (Carlo Erba, Val de Reuil, France) de concentrations croissantes. Les cellules ont ensuite été incluses en blocs d'Araldite (Fluka, st Quentin Fallavier, France). De fines sections ont ensuite été coupées à partie des blocs à l'aide d'un microtome Ultracut (Reichert-Leica, Marseille, France) puis colorées par une solution saturée d'acétate d'uranyl (Merck, Damstadt, Allemagne) en methanol et de nitrate de plomb et de citrate de sodium (Merck) en eau avant examen sur un microscope électronique Jeol 1220 (Jeol, Croissy sur Seine, France). Cet examen a permis d'observer les bactéries en localisation intracellulaire, libres dans le cytoplasme. Aucune bactérie n'a été observée dans le noyau.

### Exemple 2

### Production et caractérisation des anticorps polyclonaux de souris.

Les bactéries utilisées pour l'inoculation aux souris ont été préalablement purifiées à partir de boites de cultures cellulaires de 174 cm² à 80% d'infection. Le surnageant a été retiré des boites, les cellules incubé 45 minutes à 30°C avec de la trypsine à 0.5% (Gibco). Les cellules ont ensuite été reprises avec le surnageant puis lysées par 6 étapes de sonication (40 watts-1 min.). Les cellules résiduelles ont été ensuite retirée par centrifugation à 1500 rpm pendant 15 min. Le surnageant a été déposé sur une solution de sucrose à 25% en PBS. Après une centrifugation de 30 min. à 7500 rpm à 4° C, le culot contenant les bactéries a été repris dans 2 ml de PBS. Les bactéries ont enfin été purifiées par centrifugation à 25000 rpm pendant 1 h à 4°C dans un gradient de renograffine (45 à 25%). Après centrifugation, la couche correspondant aux bactéries a été récoltée et les bactéries rincée en PBS par 2 étapes de centrifugation à 10000rpm pendant 10 min.

Des souris âgées de 6 à 8 semaines ont été inoculées par voie intrapéritonéale à J0, J10, J20 et J30 avec 0.5 mL d'une solution à 10⁶/mL de bactéries purifiées et d'adjuvant complet de Freund. A J40, les souris ont été sacrifiées et le sang a été récolté par ponction intracardiaque. Après séparation, le sérum a été testé par immunofluorescence indirecte puis congelé à -20°C. Avant utilisation le sérum était dilué au 1 :50 et adsorbé avec les cellules XTC-2 afin de retirer les anticorps anti-souris.

### Exemple 3

### Sérodiagnostic par immunofluorescence indirecte et Western Blot

### 1- Préparation de l'antigène

*Rickettsia pulicis* (URRWFXCal2) a été cultivée sur des tapis confluents de cellules XTC-2 comme décrit précédemment. *R*. *conorii* (Moroccan strain, ATCC VR 141), *R. prowazekii* (Brein-1) and *R. typhi* (Wilmington strain, ATCC VR 144) ont été cultivées sur des tapis confluents de cellules Vero dans des boites de culture cellulaires de 174 cm² à respectivement 32°C , 35°C et 35°C. Quand 80% des cellules sont infectées, les cellules et les surnageants sont récoltés, centrifugés à 10,000 x g pendant 10 min, lavés 4 fois dans 40 ml de PBS, puis resuspendu enfin dans le plus petit volume possible d'eau distillée stérile. Le contenu final en protéine de cette solution est ensuite mesuré par spectrophotométrie ultraviolet puis ajustée à une concentration finale de 1 mg/mL avant congélation à -20°.

### 2- Immunofluorescence indirecte (IFA)

Les 4 antigènes sont ensuite déposée à l'aide d'une plume dans chaque puits d'une lame à 30 puits (Dynatech Laboratories Ltd., Billingshurt, United Kingdom), séchés a l'air puis fixé dans de l'acétone pendant for 10 min. Tous les sérums ont été dilués au 1/4, 1/8, 1/16, 1/32, 1/64 and 1/128 dans du PBS avec 3% de lait en poudre écrémé. L'immunofluorescence indirecte avec détermination des IgG et IgM après adsorption du facteur rhumatoïde a été réalisée par la technique usuellement utilisée dans le laboratoire [La Scola, B. et al. Laboratory diagnosis of rickettsioses : current approaches to diagnosis of old and new rickettsial diseases. (1997) J. Clin. Microbiol. 35 :2715-2727]. Des titres de 1/64 en IgG et/ou 1/32 en IgM ont été considérés comme positifs. En cas de réaction sérologique croisée entre plusieurs espèces de rickettsies, une réaction sérologique est considérée comme étant spécifiquement dirigée contre une espèce si la somme des titres d'IgG et d'IgM est d'au moins deux dilutions supérieure aux titres d'IgG+IgM contre la ou les espèces avec lesquelles la réaction croisée est observée.

### 3- Western blot

*R. pulicis, R.typhi* et *R.conorii* après purification ont été suspendues dans de l'eau distillée stérile et ajustées à 2 mg/mL. La procédure utilisée pour la réalisation du Western blot a été antérieurement décrite [Eremeeva, M. N., el al. Serologic response of patients suffering from primary and recrudescent typhus : comparison of complement fixation reaction, Weil-Felix test, microimmunofluorescence, and immunoblotting. Clin. Diaf. Lab.Immunol. (1995) 1 :318-324]. Les anticorps réagissant contre les antigènes de 20-50 kD sont spécifiques du LPS chez *R.typhi* et *R.conorii.* L'intensité de la réactivité des différentes bandes a été évaluée par acquisition d'image en vidéo (The Imager, Appligene, Illkirch, France).

### 4- Choix des sérums

Afin d'évaluer la séroprévalence contre *R. pulicis* dans la population générale, 100 sérums de donneurs de sang ont été testés. Afin de tester les réactions croisées entre les sérologies contre *R.conorii* et *R.typhi,* nous avons testé des sérum de phase aiguë de 97 patients ayant une fièvre boutonneuse méditerranéenne, 16 un typhus murin et 67 un typhus épidémique. Pour l'ensemble de ces patients, nous disposions de données cliniques et épidémiologiques. L'ensemble de ces sérums a été testé contre *R. pulicis, R. prowazekii, R. typhi* et *R. conorii.*

### 5- Résultats des sérologies

Aucun des 100 sérums de donneurs de sang ne présentait de sérologie positive contre *R.pulicis.*

Une réaction sérologique croisée entre *R. prowazekii* et *R*. *pulicis* a été observée pour 51 des 67 sérums de patients atteints de typhus épidémique (76.1 %). Cependant, aucun des 67 sérums ne présentait de titre d'anticorps anti-*R.pulicis* supérieur au titre d'anticorps dirigé contre *R. prowazekii,* que ce soit en IgG ou en IgM.

Une réaction sérologique croisée entre *R. typhi* et *R. pulicis* a été observée pour 11 des 16 sérums de patients atteints de typhus murin (68.7%). Cependant, aucun des 16 sérums ne présentait de titre d'anticorps anti-*R*.*pulicis* supérieur au titre d'anticorps dirigé contre *R*. *typhi,* que ce soit en IgG ou en IgM.

Une réaction sérologique croisée entre *R*. *conorii* et *R*. *pulicis* a été observée pour 67 des 97 sérums de patients atteints de fièvre boutonneuse méditerranéenne (69.0%). 96 des 97 sérums présentaient des titres d'anticorps anti-*R.pulicis* inférieurs aux titres d'anticorps dirigé contre *R. conorii,* que ce soit en IgG ou en IgM. Un patient présentait des titres d'anticorps contre *R. pulicis* plus élevés de 2 dilutions que contre *R. conorii* et était par conséquent suspect d'infection à *R*. *pulicis.* Une procédure de confirmation par Western blot a été effectuée sur ce sérum et a confirmé que ce patient présentait des anticorps spécifiquement dirigés contre *R*. *pulicis.*

### Exemple 4

### 4.1 Analyse de protéines majeures par électrophorèse en gel de polyacrylamide-dodécylsulfate de sodium (SDS-PAGE) et transfert de Western

Des souches de rickettsies ont été purifiées à la rénografine. Des préparations de protéines de *R*. *Pulicis, R*. *typhi, R*. *prowazekii, R. canadensis, R. conorii and R. akari* purifiées à la rénografine ont été remises en suspension dans du tampon d'échantillon de SDS-PAGE (Tris 0,625 M, pH 8,0, 2 % (m/v) de dodécylsulfate de sodium, 5 % (v/v) de 2-mercaptoéthanol, 10 % (v/v) de glycérol et 0,002 % (m/v) de bleu de bromophénol). Une portion aliquote de chacune d'elles a ensuite été chauffée pendant 10 min à 100°C, puis des portions aliquotes chauffées et non chauffées ont été chargées sur des gels de polyacrylamide à gradient linéaire de 9-16 % (18 cm x 20 cm x 1,5 mm). Les protéines ont été résolues par électrophorèse à 40 mA pendant 5 h à 10°C (Laemmli, 1970). Les protéines résolues ont été colorées par coloration à l'argent. Les protéines immunogènes majeures ont été étudiées par transfert de Western à l'aide d'antigènes non chauffés purifiés de la manière décrite antérieurement (Laemmli, 1970 ; Teysseire et al., 1992) et d'anticorps polyclonaux murins anti-*R*. *pulicis.*

### 4.2 Sensibilité aux antibiotiques

L'activité de l'érythromycine contre *R. Pulicis* a été déterminée dans des cellules Vero par un dosage colorimétrique au moyen de la coloration de Gimenez. Des cultures de cellules Vero dans des microplaques de titrage à 48 puits ont été infectées avec 2 000 PFU de rickettsies pendant 1 h à la température ambiante, de l'érythromycine a été ajoutée à différentes concentrations dans différentes rangées. Les rangées sans médicament infectées avec 2000, 200, 20 et 0 PFU servent de témoins. Après 9 jours d'incubation des microplaques à 32°C, des monocouches de culture cellulaire ont été colorées avec le colorant de Gimenez pour révéler la présence de foyers infectés (amas de rickettsies) dans 25 domaines choisis au hasard pour chaque puits. La concentration d'antibiotiques minimum permettant une inhibition complète de la formation de foyers par rapport à un témoin sans médicament a été notée comme CIM. Pour la doxycycline et la rifampine, une seule concentration, de 4 µg/ml, a été testée. Les expériences ont été réalisées en double pour confirmer les résultats. La température d'incubation a été abaissée à 32°C pour permettre la croissance de *R. Pulicis. R. conorii et R. typhi* ont été traités de la même manière comme témoin.

### 4.3 Résultats et discussion

Sur la figure 1, on a représenté le profil SDS-PAGE coloré à l'argent de préparations protéiques de cellules entières de *R. conorii, R. Pulicis, R. typhi ;* Piste 1, *R. conorii;* piste 2 *R*. *Pulicis ;* piste 3, *R. typhi ;* piste 4, *R. conorii;* piste 5, *R*. *pulicis ;* piste 6, *R. akari.* Des étalons de masse moléculaire sont inclus aux deux extrémités du gel.

Les pistes 1 à 3 correspondent aux antigènes froids. Les pistes 4 à 6 correspondent aux antigènes chauds.

Le profil de SDS-PAGE de *R. Pulicis* était nettement différent de ceux obtenus à partir de *R*. *typhi* (figure 1), *R. prowazekii, R. canadensis, R. conorii* (figure 2) *et R. akari.* Comme *R. conorii, R. Pulicis* exprimait une protéine de haute masse moléculaire ayant une masse moléculaire supérieure à 150 kDa, ce qui n'était pas le cas de *R. typhi et R. prowazekii. R. Pulicis* exprimait aussi une protéine thermolabile de 30 kDa qui n'était pas observée dans les profils d'autres rickettsies étudiées. Ainsi, le profil protéique en SDS-PAGE de la souche selon la présente invention est nettement différent de celui de *"R. felis"* dont on a constaté antérieurement qu'il ressemblait étroitement à celui de *R. typhi* concernant le transfert de Western (Radulovic et al., 1995b ; Higgins et al., 1996 ; Azad et al., 1997), tandis qu'il a été démontré que les antigènes immunogènes de R. Publicis étaient discernables de ceux de *R. typhi* dans un sérum humain réagissant spécifiquement à *R. pulicis* et dans des antisérums murins produits contre l'isolat purifié, du fait d'une réaction contre l'antigène de 30 kDa.

La fluorescence et la double coloration d'actine et de bactéries ont été réalisées pour évaluer l'aptitude de *R. pulicis* à polymériser l'actine intracellulairement. La polymérisation polaire de l'actine n'a pas été observée dans des cellules infectées par *R. pulicis* ou *R*. *typhi* contrairement à celles infectées par *R. conorii.* Il semble que la polymérisation de l'actine est associée à l'aptitude à croître à l'intérieur du noyau, une caractéristique commune à des rickettsies du groupe de la fièvre tachetée étudiées jusqu'à maintenant, mais absente dans le groupe du typhus et *R*. *Pulicis* (Heinzen et al., 1993 ; Teysseire et al., 1992 ; Burgdorfer et al., 1968).

Pour *R. Pulicis,* la CIM pour l'érythromycine était 32 µg/ml, et une concentration de 4 µg/ml de doxycycline ou de rifampine était inhibitrice. Les résultats obtenus pour les témoins étaient identiques à ceux obtenus antérieurement (Rolain et al., 1998). L'isolat de *R*. *Pulicis* est résistant à l'érythromycine, tandis que la sensibilité à cet antibiotique était une caractéristique limitée aux rickettsies du groupe du typhus (Rolain et al., 1998) et à la bactérie appelée *"R. felis"* (Radulovic et al., 1995a ; Radulovic et al., 1996).

Les gènes *rOmpA* et *rpoB* ont été séquencés de la base 3539 à la base 6722 et de la base 1 à la base 3866 respectivement. Pour *rpoB,* les similitudes de séquences étaient de 96 % (*R. massiliae,* Bar 29, *R. conorii*) à 87 % pour les séquences nucléotidiques. La similitude de séquence était de 92 % entre *R. pulicis* et *R. prowazekii.* Pour les séquences d'acides aminés, les similitudes étaient de 98 % (*R. massiliae,* Bar 29, *R. conorii*) à 83 % (*R. typhi*)*.* La similitude de séquence était de 96 % entre R. pulicis et R. prowazekii. Les dendrogrammes obtenus à partir de *rOmpA* avec les trois méthodes de construction d'arbre différentes utilisées présentaient une position phylogénétique similaire pour *R*. *pulicis.* Ils formaient des amas avec *R*. *australis* avec une valeur de sangle de botte de 100 %.

Des données additionnelles qui peuvent différencier la souche *R. pulicis* de la souche *"R. felis"* maintenant perdue sont la température de croissance. *R. pulicis* ne poussait pas à 35 ou 37°C, tandis qu'il a été décrit que *"R. felis"* induisait un effet cytopathique conduisant à la formation de petites plages tardives quand elle était cultivée à 34°C dans des cellules Vero, des HUVEC ou des cellules L929 (Radulovic et al., 1995b). De plus, même à 28°C, *R. pulicis* n'avait pas la capacité de croître sur des cellules L929. Ces résultats démontrent que l'isolat de *"R. felis"* maintenant perdu n'était pas le même organisme que celui récupéré de manière répétée à partir de puces de chats infectées obtenues auprès de la colonie de El Laboratory. La description phénotypique de l'isolat de *R. Pulicis* est nettement différente de celle de l'isolat de *"R. felis"* original. De plus, les inventeurs ont été capables d'isoler seulement *R*. *typhi* à partir de stocks de la souche "*R*. *felis"* originale qui ont été fournis à leur laboratoire après son isolement décrit.

| Caractère | *R. conorii* | *R. typhi* | *R. pulicis* | *"R. felis*" |
|---|---|---|---|---|
| Croissance possible à: | | | | |
| 28°C | + | + | + | NF |
| 32°C | + | + | + (faible) | NF |
| 34°C | + | + | - | + |
| Croissance possible sur: | | | | |
| cellules XTC-2 | + | + | + | NF |
| cellules Vero | + | + | + | + |
| cellules L-929 | + | + | - | + |
| Localisation dans le: | | | | |
| cytoplasme | + | + | + | + |
| noyau | + | - | - | - |
| Polymérisation de l'actine | + | - | - | NF |
| Inhibition par 4µg/ml de: | | | | |
| Doxycycline | + | + | + | + |
| Rifampin | + | + | + | + |
| Erythromycin | - | + | - | + |
| Proteines sur SDS-PAGE | | | | |
| 150 kDa | + | - | + | - |
| 30 kDa | - | - | + | - |
| 17 kDa | + | + | + | + |
| Gènes détectés | | | | |
| *OmpA* | + | - | + | - |
| *OmpB* | + | + | + | + |

Adams, J.R., Schmidtmann, E.T., & Azad, A.F. (1990). Infection of colonized cat fleas, Ctenocephalides felis (Bouché), with a rickettsia-like microorganism. Am J Trop Med Hyg 43, 400-409.
Azad, A.F., Sacci, J.B., Nelson, W.M., Dasch, G.A., Schmidtmann, E.T., & Carl, M. (1992). Genetic characterization and transovarial transmission of a typhus-like rickettsia found in cat fleas. Proc Natl Acad Sci USA 89, 43-46.
Azad, A.F., Radulovic, S., Higgins, J.A., Noden, B.H., & Troyer, J.M. (1997). Flea-borne rickettsioses : ecologic considerations. Emerg Infect Dis 3, 319-327.
Bouyer, D.H., Crocquet-Valdes, P.A., & Walker, D.H. (2000). Expression and size determination of the rOmpA protein of Rickettsia felis. In American Society for Rickettsiology - 15th meeting. pp. 61-61.
Burgdorfer, W., Anacker, R.L., Bird, R.G., & Bertram, D.S. (1968). Intranuclear growth of Rickettsia rickettsii. J Bacteriol 96, 1415-1418.
Drancourt, M., Raoult, D. (1999). Characterization of mutations in rpoB gene in naturally rifampin-resistant Rickettsia species. Antimicrob Agents Chemother 43, 2400-2403.
Fournier, P.E., Roux, V., Raoult, D. (1998). Phylogenetic analysis of spotted fever group rickettsiae by study of the surface protein rOmpA. Int J Syst Bacteriol 48, 839-849.
Heinzen, R.A., Hayes, S.F., Peacock, M.G., & Hackstad, T. (1993). Directional actin polymerization associated with spotted fever group rickettsia infection of Vero cells. Infect Immun 61, 1926-1935.
Higgins, J.A., Radulovic, S., Schriefer, M.E., & Azad, A.F. (1996). Rickettsia felis : a new species of pathogenic rickettsia isolated from cat fleas. J Clin Microbiol 34, 671-674.
La Scola, B., & Raoult, D. (1996). Diagnosis of Mediterranean spotted fever by cultivation of Rickettsia conorii from blood and skin samples using the centrifugation-shell vial technique and by detection of R. conorii in circulating endothelial cells: a 6 year follow-up. J Clin Microbiol 34, 2722-2727.
Laemmli, U.K. (1970). Cleavage of structural proteins during the assembly of the head of bacteriophage T4. Nature 227, 680-685.
Murray, R.G.E., & Schleifer, K.H. (1994). Taxonomic notes : a proposal for recording the properties of putative taxa of procaryotes. Int J Syst Bacteriol 44, 174-176.
Noden, B.H., Radulovic, S., Higgins, J.A., & Azad, A.F. (1998). Molecular identification of Rickettsia typhi and R.felis in co-infected Ctenocephalides felis (Siphonaptera: Pulicidae). J Med Entomol 35, 410-414.
Radulovic, S., Higgins, J.A., Jaworski, D.C., & Azad, A.F. (1995a). In vitro and in vivo antibiotic susceptibilities of ELB rickettsiae. Antimicrob Agents Chemother 39, 2564-2566.
Radulovic, S., Higgins, J.A., Jaworski, D.C., Dasch, G.A., & Azad, A.F. (1995b). Isolation, cultivation, and partial characterization of the ELB agent associated with cat fleas. Infect Immun 63, 4826-4829.
Radulovic, S., Higgins, J.A., Jaworski, D.C., & Azad, A.F. (1996). In vitro and in vivo antibiotic susceptibilities of ELB rickettsiae (vol 39, pg 2564, 1996). Antimicrob.Agents Chemother., 40, 2912.
Rolain, J.M., Maurin, M., Vestris, G., & Raoult, D. (1998). In vitro susceptibilities of 27 Rickettsiae to 13 antimicrobials. Antimicrob Agents Chemother 42, 1537-1541.
Teysseire, N., Chiche-Portiche, C., & Raoult, D. (1992). Intracellular movements of Rickettsia conorii and R. typhi based on actin polymerization. Res Microbiol 143, 821-829.

## Revendications

1. Culture cellulaire d'une bactérie *Rickettsia* correspondant à l'agent ELB, isolée et établie en culture de manière reproductible pouvant être obtenue par une méthode dans laquelle :
1) on réalise une culture de cellules de préférence de cellules de *Xenopus laevis,* à la température de culture de 25 à 30°C de préférence 28°C, et
2) les bactéries sont inoculées par centrifugation du broyat de puces ou tout autre prélèvement d'origine humaine ou animale infecté par lesdites bactéries sur la couche de cellules en culture de l'étape 1 et incubation à 25 à 30°C de préférence 28°C jusqu'à détection d'une croissance bactérienne,
3) les bactéries sont établies en culture par repiquages successifs sur des cellules en culture.

2. Culture cellulaire de la bactérie *Rickettsia* correspondant à l'agent ELB selon la revendication 1, déposée à la CNCM de l'Institut Pasteur, le 8 décembre 1999 sous le n° de réception I-2363.

3. Culture cellulaire selon la revendication 1 ou 2, **caractérisée en ce que** les cellules sont des cellules de *Xenopus laevis* infectées par ladite bactérie, lesdites cellules étant obtenues à partir des cellules saines XTC-2 déposées à la CNCM de l'Institut Pasteur le 8 décembre 1999 sous le n° d'enregistrement I-2364.

4. Bactérie *Rickettsia* correspondant à l'agent ELB isolée et purifiée après centrifugation du surnageant d'une culture cellulaire selon l'une des revendications 1 à 3.

5. Utilisation d'une bactérie selon la revendication 4 ou d'une culture suivant l'une des revendications 1 à 4 pour le diagnostic in-vitro de maladies liées à des infections par ladite bactérie.

6. Méthode de diagnostic sérologique in vitro d'une infection par la bactérie *Rickettsia* correspondant à l'agent ELB, **caractérisée en ce que** l'on met en contact une bactérie selon la revendication 4, ou une culture selon l'une des revendications 1 à 3 avec un échantillon provenant du patient consistant dans un sérum, un fluide biologique ou un prélèvement humain, et l'on détecte une réaction immunologique.

7. Méthode selon la revendication 6 pour le diagnostic sérologique in vitro à *Rickettsia* qui comprend l'étape consistant essentiellement à détecter une réaction immunologique entre un anticorps spécifique d'une immunoglobuline humaine reconnaissant la bactérie selon la revendication 4 et une immunoglobuline humaine reconnaissant ladite bactérie selon la revendication 4.

8. Méthode selon la revendication 7, **caractérisée en ce que** ledit anticorps spécifique d'une immunoglobuline humaine, de préférence de type G, M ou A, est une immunoglobuline anti humaine animale, de préférence de chèvre.

9. Méthode de diagnostic sérologique selon l'une des revendications 6 à 8 qui comprend les étapes suivantes :
- le dépôt dans ou sur un support solide de solution contenant la bactérie tel qu'elle est définie dans la revendication 4,
- l'introduction dans ou sur le dit support du sérum ou du fluide biologique à tester,
- l'introduction dans ou sur le support d'une solution d'un anticorps marqué spécifique d'une immunoglobuline humaine reconnaissant ladite bactérie,
- l'observation d'une période d'incubation,
- le rinçage du support solide, et
- la détection de ladite réaction immunologique.

10. Méthode selon la revendication 9 dans lequel l'anticorps marqué est marqué par une sonde radioactive d'une enzyme ou d'un agent fluorescent.

11. Méthode selon la revendication 10, dans lequel l'agent fluorescent est l'isothiocyanate de fluorescéine

12. Méthode selon une des revendications 9 à 11 dans lesquelles on utilise 0,5 à 5 µl de préférence environ 1 µl de la dite solution contenant ladite bactérie.

13. Dispositif pour la mise en oeuvre de la méthode selon l'une des revendications 9 à 12 qui comprend un support solide dans ou sur lequel la solution comprenant ladite bactérie était disposée.

14. Dispositif selon la revendication 13 selon laquelle le support solide est une lamelle de verre.

15. Trousse pour la détection in vitro de la bactérie *Rickettsia* correspondant à l'agent ELB selon la méthode d'une des revendications 6 à 12 comprenant essentiellement comme composants :
- une solution contenant la bactérie ou une culture cellulaire telle que définie dans l'une des revendications 1 à 4, et de préférence,
- une solution contenant un anticorps spécifique d'une immunoglobuline humaine reconnaissant ladite bactérie selon la revendication 4.

16. Trousse suivant la revendication 15 **caractérisée en ce que** ledit anticorps spécifique est marqué.

17. Trousse suivant la revendication 16 **caractérisée en ce que** ledit anticorps spécifique est marqué par un isotope radioactif, une enzyme ou un composé fluorescent.

## Claims

1. A cell culture of Rickettsia bacterium corresponding to the ELB agent, isolated and established in culture in a reproducible manner and that can be obtained by a method in which:
1) a cell culture is carried out preferably with Xenopus laevis cells, at a culture temperature between 25 and 30°C, preferably at 28°C, and
2) bacteria are inoculated by centrifugation of ground fleas, or any other sample from a human or animal source infected by said bacteria, on a layer of the cells in culture from step 1, and incubated between 25 and 30°C, preferable at 28°C, until bacterial growth is observed,
3) bacteria are established in culture by successive re-inoculations on cultured cells.

2. A cell culture of Rickettsia bacterium corresponding to the ELB agent, according to claim 1, filed with CNCM (National Collection of Microorganism Cultures at the Institut Pasteur, France) on 8 December 1999 under receipt No. 1-2363.

3. A cell culture according to claim 1 or claim 2, **characterised in that** the cells are Xenopus laevis cells infected with said bacterium, said cells being obtained from healthy XTC-2 cells, filed at the Institut Pasteur's CNCM on 8 December 1999 under registration number 1-2364.

4. Rickettsia bacterium corresponding to the ELB agent isolated and purified after centrifuging the supernatant of a cell culture according to one of claims 1 to 3.

5. Use of a bacterium according to claim 4 or of a culture according to one of claims 1 to 4 for in vitro diagnosis of diseases related to infection by said bacterium.

6. Method for in vitro serological diagnosis related to infection by the Rickettsia bacterium corresponding to the ELB agent, **characterised in that** a bacterium according to claim 4, or a culture according to one of claims 1 to 3 is contacted with a sample originating from a patient consisting of serum, biological fluid, or a human sample, and an immunological reaction is detected.

7. Method according to claim 6 for in vitro serological diagnosis of Rickettsia that includes a step which basically consists in detecting an immunological reaction between a specific antibody of a human immunoglobulin which recognizes said bacterium according to claim 4 and a human immunoglobulin which recognizes said bacterium according to claim 4.

8. Method according to claim 7, **characterised in that** said specific antibody of a human immunoglobulin, preferably of the G, M or A type, is an animal immunoglobulin, preferably a goat immunoglobulin.

9. Serological diagnosis method according to one of claims 6 to 8 which includes the following steps:
- depositing a solution of the bacterium as defined in claim 4 in or onto a solid support,
- adding the serum or biological fluid to be tested in or onto said support,
- adding a specific labelled antibody solution of a human immunoglobulin which recognizes said bacterium in or onto the support,
- incubating for a fixed period of time,
- rinsing the solid support, and
- carrying out detection of said immunological reaction.

10. Method according to claim 9 in which the labelled antibody is labelled with a radioactive probe of an enzyme or fluorescent agent.

11. Method according to claim 10 in which the fluorescent agent is fluroesceine isothiocyanate.

12. Method according to one of claims 9 to 11 in which 0.5 to 5 µl, preferably about 1 µl, of said solution containing said bacterium is used.

13. Device for application of the method according to one of claims 9 to 12 which includes a solid support into or onto which the solution containing the bacterium is deposited.

14. Device according to claim 13 whereby the solid support is a glass slide.

15. Kit for in vitro detection of the Rickettsia bacterium corresponding to the ELB agent, according to the method of one of claims 6 to 12 which consists of the following basic elements:
- a solution containing the bacterium or cell culture as defined in one of claims 1 to 4, and preferably,
- a solution containing a specific antibody of a human immunoglobulin which recognizes said bacterium according to claim 4.

16. Kit according to claim 15 **characterised in that** said specific antibody is labelled.

17. Kit according to claim 16 **characterised in that** said specific antibody is labelled with a radioactive isotope, an enzyme or a fluorescent compound.

## Patentansprüche

1. Zellkultur eines *Rickettsia*-Bakteriums, das der ELB-Substanz entspricht, isoliert und etabliert in Kultur in reproduzierbarer Weise, welche erhältlich ist durch ein Verfahren, in dem:
1) man eine Zellkultur vorzugsweise von Zellen von *Xenopus laevis* bei der Kulturtemperatur von 25 bis 30°C, vorzugsweise 28°C ausführt, und
2) die Bakterien angeimpft werden durch Zentrifugation der Zerkleinerung von Flöhen oder jeder anderen Probennahme menschlichen oder tierischen Ursprungs, infiziert durch die Bakterien auf der Zellkulturschicht des Schrittes 1) und Inkubation bei 25 bis 30°C, vorzugsweise 28°C bis zur Detektion eines Bakterienwachstums,
3) die Bakterien in Kultur etabliert werden durch aufeinander folgendes Verpflanzen auf den Zellen in Kultur.

2. Zellkultur des Bakteriums *Rickettsia,* das der ELB-Substanz entspricht, gemäß Anspruch 1, hinterlegt bei der CNCM des Institut Pasteur am 8. Dezember 1999 unter der Hinterlegungsnummer I-2363.

3. Zellkultur nach Anspruch 1 oder 2, **dadurch gekennzeichnet, daß** die Zellen Zellen von *Xenopus laevis* sind, die infiziert sind mit dem Bakterium, wobei die Zellen erhalten sind aus gesunden XTC-2-Zellen, hinterlegt bei der CNCM des Institut Pasteur am 8. Dezember 1999 unter der Hinterlegungsnummer I-2364.

4. *Rickettsia*-Bakterium, das der ELB-Substanz entspricht, isoliert und gereinigt nach Zentrifugation des Überstands einer Zellkultur gemäß einem der Ansprüche 1 bis 3.

5. Verwendung eines Bakteriums nach Anspruch 4 oder einer Kultur nach einem der Ansprüche 1 bis 4 für die In-vitro-Diagnose von Krankheiten, die verbunden sind mit Infektionen durch das Bakterium.

6. Serologisches In-vitro-Diagnoseverfahren einer Infektion durch das Bakterium *Rickettsia,* das der ELB-Substanz entspricht, **dadurch gekennzeichnet, daß** man ein Bakterium nach Anspruch 4 oder eine Kultur nach einem der Ansprüche 1 bis 3 mit einer Probe kontaktiert, die vom konsistenten Patienten kommt und aus einem Serum, einer biologischen Flüssigkeit oder einer menschlichen Probenentnahme besteht, und man eine immunologische Reaktion detektiert.

7. Verfahren nach Anspruch 6 zur serologischen In-vitro-Diagnose auf *Rickettsia,* das den Schritt umfaßt, der im wesentlichen darin besteht, eine immunologische Reaktion zwischen einem spezifischen Antikörper, der das Bakterium gemäß Anspruch 4 erkennt und einem menschlichen Immunoglobulin, das das Bakterium gemäß Anspruch 4 erkennt, zu detektieren.

8. Verfahren nach Anspruch 7, **dadurch gekennzeichnet, daß** der spezifische Antikörper eines menschlichen Immunoglobulins, vorzugsweise vom Typ G, M oder A, ein tierisches Antihumanimmunoglobulin ist, vorzugsweise von der Ziege.

9. Verfahren zur serologischen Diagnose gemäß einem der Ansprüche 6 bis 8, das die folgenden Schritte umfaßt:
- Abscheidung in oder auf einem festen Träger einer Lösung, die das Bakterium wie in Anspruch 4 definiert, enthält,
- Einführung in oder auf den Träger von dem Serum oder biologischen Fluid, das zu testen ist,
- Einführung in oder auf den Träger einer Lösung eines spezifischen markierten Antikörpers eines humanen Immunoglobulins, das das Bakterium erkennt,
- Beobachtung eines Inkubationszeitraums,
- Spülen des festen Trägers und
- Detektion der immunologischen Reaktion.

10. Verfahren nach Anspruch 9, bei dem der markierte Antikörper durch eine radioaktive Sonde eines Enzyms oder eines Fluoreszenzmittels markiert ist.

11. Verfahren nach Anspruch 10, bei dem das Fluoreszenzmittel Fluoreszeinisothiocyanat ist.

12. Verfahren nach einem der Ansprüche 9 bis 11, bei dem man 0,5 bis 5 µl, vorzugsweise etwa 1 µl der das Bakterium enthaltenden Lösung verwendet.

13. Vorrichtung zur Durchführung des Verfahrens nach einem der Ansprüche 9 bis 12, die einen festen Träger umfaßt, in oder auf dem die Lösung, die das Bakterium umfaßt, abgeschieden wurde.

14. Vorrichtung nach Anspruch 13, bei welcher der Träger eine Glaslamelle ist.

15. Kit zur In-vitro-Detektion des Bakteriums *Rickettsia,* das der ELB-Substanz entspricht, gemäß dem Verfahren nach einem der Ansprüche 6 bis 12, im wesentlichen als Bestandteile umfassend:
- eine Lösung, die das Bakterium oder eine Zellkultur wie definiert in einem der Ansprüche 1 bis 4 enthält, und vorzugsweise
- eine Lösung, die einen spezifischen Antikörper eines menschlichen Immunoglobulins enthält, welches das Bakterium gemäß Anspruch 4 erkennt.

16. Kit nach Anspruch 15, **dadurch gekennzeichnet, daß** der spezifische Antikörper markiert ist.

17. Kit nach Anspruch 16, **dadurch gekennzeichnet, daß** der spezifische Antikörper markiert ist durch ein radioaktives Isotop, ein Enzym oder eine Fluoreszenzverbindung.
